# FASCICULE DE BREVET EUROPEEN

(11) **EP 0 610 124 B1**
(45) Date de publication et mention de la délivrance du brevet: **03.09.1997**
(21) Numéro de dépôt: 94400200.5
(22) Date de dépôt: 31.01.1994
(51) Int. Cl.: A61F 2/76

(54) **Système d'assemblage de deux constituants d'une prothèse**
Verbindungssystem für zwei Teile einer Prothese
Assembly system of two prosthesis parts

(30) Priorité: 03.02.1993 FR 9301156; 28.07.1993 FR 9309297
(43) Date de publication de la demande: 10.08.1994
(73) Titulaire: ETABLISSEMENTS PROTEOR Société anonyme dite:, 21100 Dijon (FR)
(72) Inventeur: Vera, Bernard, F-21250 Seurre (FR); Pierron, Olivier, L-1210 Luxembourg (LU)
(74) Mandataire: Jolly, Jean-Pierre

(56) Documents cités:
- EP-A- 0 102 853
- EP-A- 0 224 373
- GB-A- 2 141 345

## Description

La présente invention concerne un système d'assemblage, en une pluralité de positions réglables, de deux constituants d'une prothèse.

On sait que l'alignement et l'orientation de parties d'une prothèse doivent être réalisés avec soin et l'on utilise souvent dans ce but des pièces de jonction relativement compliquées et coûteuses.

On a déjà proposé d'utiliser des organes de jonction modulaires, destinés à faire partie d'une prothèse d'un membre du corps humain et à relier entre eux, de façon ajustable, deux composants de cette prothèse.

C'est ainsi que GB-A-2 141 345 décrit un dispositif de liaison ajustable pour réunir deux parties d'une prothèse, ce dispositif comprenant un premier et un second éléments de liaison destinés à être fixés chacun sur les parties de la prothèse à réunir, les éléments de liaison étant en contact mutuel ou avec un organe intermédiaire de liaison par des parties planes ou sphériques prenant directement appui les unes contre les autres et susceptibles d'être déplacées les uns par rapport aux autres par glissement ou par pivotement, des vis étant prévues pour les verrouiller en position après réglage.

Dans l'une des formes de réalisation décrites dans ce document antérieur, un organe en saillie, solidaire d'une partie de la prothèse, est logé dans une portion évidée en forme de cuvette d'un organe intermédiaire de liaison et prend appui par une partie plane contre le fond de la partie en forme de cuvette, contre laquelle il peut ainsi glisser pour être amené par translation de la position désirée. Des vis engagées dans des taraudages disposés transversalement dans les parois latérales de la cuvette peuvent prendre appui par une extrémité libre contre les flancs latéraux de l'organe en saillie, inclinés par rapport au fond de la cuvette, en vue de verrouiller cet organe en position.

Du fait du contact direct entre le fond de la cuvette et la partie en saillie, des couples très importants s'exercent sur cette dernière, qui risque donc de se rompre ou de se fissurer et qui doit, par conséquent, être réalisée en un matériau apte à supporter les efforts qui lui sont appliqués.

La présente invention vise à remédier à cet inconvénient en proposant un système d'assemblage, en une pluralité de positions, réglables en translation, en rotation et angulairement, de deux constituants d'une prothèse, dans lequel une partie en saillie solidaire de l'un de ces constituants est, comme précédemment, engagée dans un évidement formant cuvette de l'autre constituant de la prothèse ou d'un élément intermédiaire de liaison (ou inversement), tandis que des vis disposées transversalement par rapport aux parois latérales de la cuvette viennent en butée, comme dans la technique antérieure, contre les flancs latéraux de la partie en saillie, mais sans que le fond de la cuvette prenne appui directement contre la surface en regard de la partie en saillie, en évitant ainsi à celle-ci d'être soumise à des efforts ou à des couples très importants.

A cet effet, conformément à la présente invention, le bord extérieur de l'évidement formant cuvette prend appui de manière à pouvoir glisser de façon rectiligne dans au moins une direction sur une surface du constituant dont est solidaire la partie en saillie, cette partie en saillie étant en outre, en toute position, écartée du fond de l'évidement formant cuvette.

Lorsque, dans cette définition de l'invention, on parle d'un évidement formant cuvette, on n'entend naturellement pas se limiter à une forme précise de cuvette. Il en est de même de la partie en saillie, qui peut avoir n'importe quelle forme se prêtant à une verrouillage par des vis venant en butée contre ses flancs latéraux.

Dans la pratique, c'est donc par les bords de la cuvette que la partie dans laquelle cet évidement est ménagé prend appui, de manière à pouvoir glisser, contre une surface d'un élément solidaire de la base de la partie en saillie et les efforts qu'exercera la portion dans laquelle est ménagée la cuvette ne sont donc pas transmis à la partie en saillie, mais se répartissent en couronne autour de celle-ci sur l'élément qui en est solidaire, en évitant donc les risques de rupture ou de détérioration de la partie en saillie.

Par glissement relatif de l'élément solidaire de la base de la partie en saillie et des bords de l'évidement formant cuvette, il sera ainsi possible de régler à volonté en translation, la position des deux parties d'une prothèse, ou d'une partie d'une prothèse et d'un élément intermédiaire de liaison entre cette partie et une autre partie de la prothèse.

Le bord de la cuvette pourra prendre appui par au moins une première surface plane contre au moins une seconde surface plane de l'élément solidaire de la partie en saillie et, dans une telle forme de mise en oeuvre de l'invention, par glissement relatif de la première surface plane contre la seconde surface plane, on pourra régler en translation en toute direction la position des deux parties d'une prothèse ou d'une partie d'une prothèse et d'un élément intermédiaire de liaison.

Le bord de la cuvette pourra également prendre appui par au moins une première surface cylindrique contre au mois une seconde surface cylindrique de profil complémentaire de l'élément dont est solidaire la partie en saillie et, dans un telle forme de réalisation, la première et la seconde surface pouront glisser l'une par rapport à l'autre dans une direction, parallèlement à l'axe des surfaces cylindriques et pivoter l'une par rapport à l'autre autour de l'axe de la surface cylindrique, pour permettre le réglage de leurs positions relatives.

Les différents éléments pourront enfin avantageusement être réglables les uns par rapport aux autres par rotation autour d'un axe perpendiculaire aux surfaces planes de glissement ou à l'axe des surfaces cylindriques de glissement.

Dans les formes de réalisations qui seront décrites ci-après, en référence aux dessins annexés, les parties en saillie auront la forme d'un tronc de pyramide à base rectangulaire et elles feront saillie à partir d'une partie cylindrique à laquelle la partie en saillie sera raccordée par la petite base du tronc de pyramide. Ces formes de mise en oeuvre de l'invention sont des formes préférées, au fait de leur simplicité, mais elle n'ont pas de caractère limitatif.

Une forme de réalisation de l'invention est donc constituée par un dispositif modulaire de liaison, réglable en position, entre deux constituants d'une prothèse, ce dispositif comprenant un premier élément, apte à être rendu solidaire d'un premier constituant de la prothèse, un second élément, apte à être rendu solidaire d'un second constituant de la prothèse, et un élément intermédiaire, apte à être rendu solidaire, en une position réglable, respectivement, du premier et du second éléments, par coopération d'une partie mâle et/ou d'une partie femelle, respectivement, de cet élément intermédiaire, avec une partie femelle et/ou une partie mâle du premier et du deuxième éléments, des organes d'assemblage tels que des vis étant prévus pour verrouiller l'un par rapport à l'autre, dans une pluralité de positions, d'une part, le premier élément et l'élément intermédiaire, d'autre part, le second élément et l'élément intermédiaire, ce dispositif étant caractérisé en ce que chacune des dites parties mâles comprend une portion en forme de tronc de pyramide à base rectangulaire faisant saillie à partir d'une portion de forme cylindrique, à laquelle il est raccordé par sa petite base, l'axe de la portion cylindrique étant sensiblement parallèle à deux côtés de la base du tronc de pyramide associé, tandis que chaque partie femelle comprend une portion de forme complémentaire de celle de l'élément cylindrique et une portion évidée en forme de rainure apte à recevoir la portion de l'élément mâle associé en forme de tronc de pyramide, la rainure ayant des dimensions supérieures à celles de la portion en forme de tronc de pyramide, parallèlement à l'axe de la portion cylindrique et perpendiculairement à celle-ci, de manière à offrir une double liberté de mouvement, en rotation par rapport à cet axe et en translation parallèlement à cet axe, de la portion mâle par rapport à la portion femelle associée, les parties cylindriques associées à chacun des premier et second éléments ayant en outre des axes non parallèles et, de préférence, perpendiculaires entre eux.

Grâce à ce dispositif de liaison, il est donc possible, comme indiqué ci-dessus, de faire pivoter chacune des portions cylindriques mâles par rapport à la portion femelle correspondante et de déplacer en translation chacune des portions mâles en forme de tronc de pyramide par rapport à la rainure de la partie femelle associée dans laquelle elle est logée, ce qui offre ainsi deux possibilités de réglage angulaire des constituants de la prothèse l'un par rapport à l'autre et deux possibilités de réglage en translation.

Dans la définition de l'invention donnée ci-dessus, le terme forme cylindrique englobe toutes les surfaces permettant aux parties femelles et aux parties mâles associées de pivoter l'une par rapport à l'autre autour d'un axe commun de rotation.

L'élément intermédiaire du dispositif de liaison pourra comporter deux parties mâles associées chacune à une partie femelle des premier et deuxième éléments du dispositif. Inversement, l'élément intermédiaire pourra comporter deux parties femelles, associées chacune à une partie mâle des premier et deuxième éléments du dispositif de liaison. Enfin, en variante, l'élément intermédiaire pourra comporter une unique partie mâle, associée à une partie femelle de l'un des premier et deuxième éléments, et une unique partie femelle, associée à une partie mâle de l'un des premier et deuxième éléments du dispositif.

Des vis, vissées dans des évidements filetés des premier et deuxième éléments du dispositif et venant en butée contre les flancs des portions en forme de tronc de pyramide, permettent, après réglage des positions relatives des constituants de la prothèse respectivement solidaires des premier et deuxième éléments du dispositif, de verrouiller ces éléments dans leurs positions respectives.

L'invention va être décrite ci-après plus en détail, en référence aux dessins schématiques annexés, sur lesquels :
La figure 1 est une vue partielle en perspective de deux parties d'une prothèse ou d'une partie d'une prothèse et d'une partie d'un élément intermédiaire de liaison, en contact mutuel par des surfaces coplanaires ;
Les figures 2 et 3 sont des coupes, respectivement, suivant les lignes II-II et III-III de la figure 1 ;
La figure 4 est une vue schématique en perspective d'une autre forme de réalisation du dispositif conforme à l'invention ;
Les figures 5 et 6 sont des vues en coupe selon les lignes V-V et VI-VI de la figure 1 ;
Les figures 7 et 8 sont des vues en perspective schématiques de deux autre formes de mise en oeuvre de l'invention ;
Les figures 9, 10 et 11, sont des vues en perspective schématiques, respectivement, de variantes de réalisation des dispositifs des figures 4, 7 et 1.

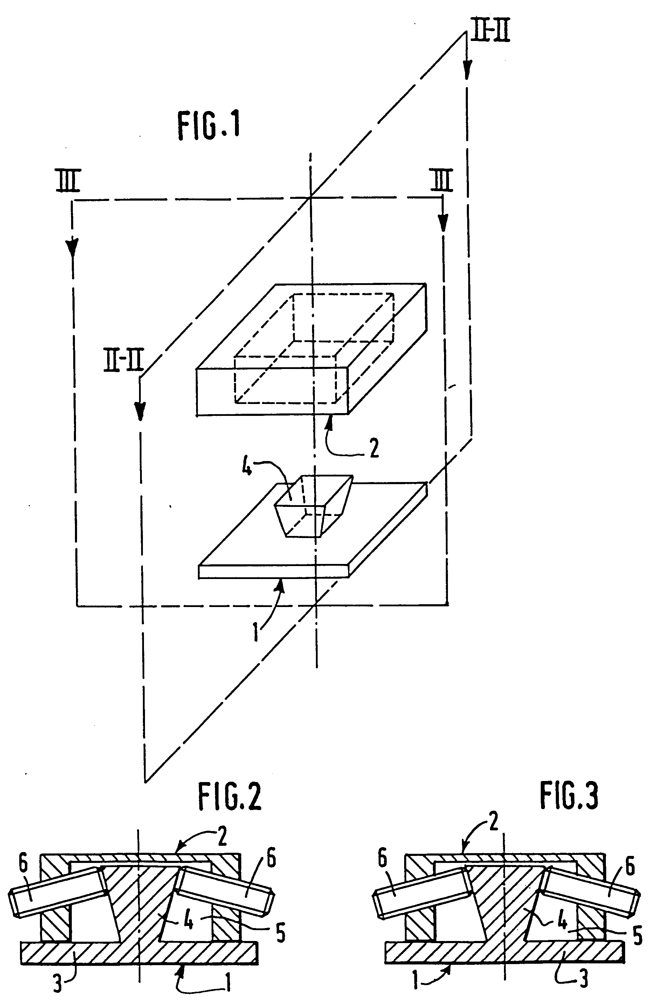

On se référera d'abord aux figures 1 à 3.

Pour plus de clarté et pour mieux faire ressortir les avantages du système conforme à l'invention, ces figures peuvent être rapprochées de la figure 3 de GB-A-2 141 345.

On voit que le système conforme à l'invention comprend deux pièces 1 et 2, qui peuvent chacune être solidaires d'une partie d'une prothèse ou d'un élément de liaison entre deux parties d'une prothèse, mais qui peuvent aussi faire partie intégrante de cette partie de prothèse ou de cette partie de liaison.

La pièce 1 comprend une plaque plane 3, à la surface de laquelle fait saillie une partie 4, qui a ici la forme d'un tronc de pyramide inversé, c'est-à-dire d'un tronc de pyramide dont la plus grande base est éloignée de la plaque 3, mais qui pourrait avoir n'importe quelle autre forme. Dans le cas du dessin, la pièce 1 est représentée comme étant monobloc, mais la plaque 3 et la partie 4 pourraient aussi bien être distinctes et rendues solidaires l'une de l'autre par tout moyen connu dans la technique.

La pièce 2 peut avoir n'importe quelle forme. Elle comprend un évidement formant cuvette 5, dont les bords reposent par une partie plane contre la surface de la plaque 3 à partir de laquelle fait saillie la partie 4. Cette dernière est engagée dans l'évidement 5 formant cuvette, mais sans venir en contact avec le fond de celle-ci, contrairement aux systèmes de la technique antérieure.

De façon connue en soi, des vis engagées dans des évidements transversaux filetés des parois latérales de la cuvette 5 peuvent venir en butée contre les flancs inclinés de la partie 4, pour immobiliser celle-ci en position et, par conséquent, pour verrouiller la partie 1 par rapport à la partie 2.

Lorsque les vis 6 ne maintiennent pas assemblées les pièces 1 et 2, il est possible de faire glisser ces pièces l'une contre l'autre en toute direction pour régler leur position relative en translation.

La pièce 2 ne prenant pas appui contre la partie en saillie 4, celle-ci ne supporte aucun effort et ne risque pas de se briser, de se fissurer ou de se détériorer, comme dans la technique antérieure. Au contraire, les sollicitations de la partie 2 s'exercent contre la surface de la plaque 3, tout autour de la partie 4, et sont ainsi réparties sur toute cette surface, qui les supporte sans aucun problème.

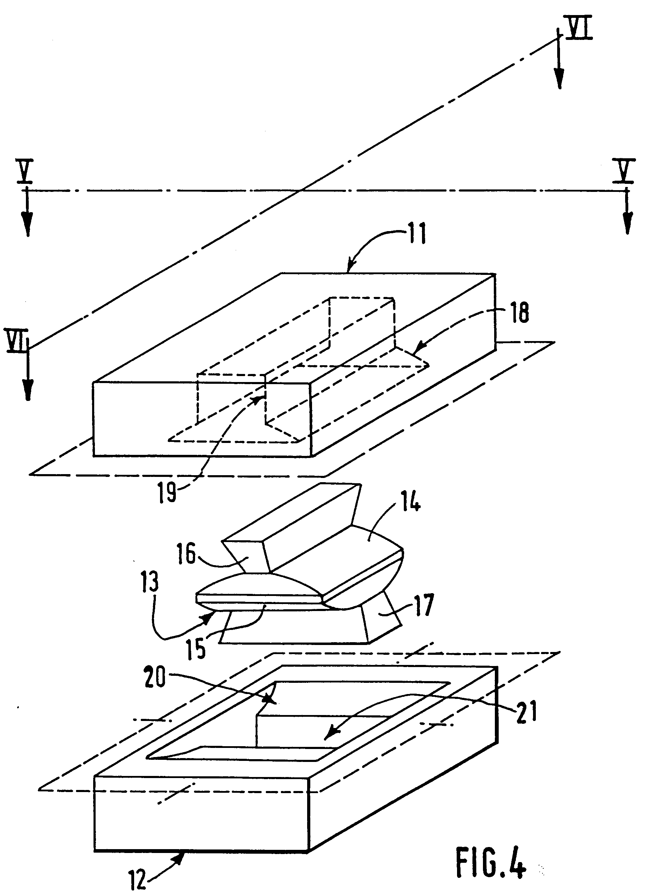

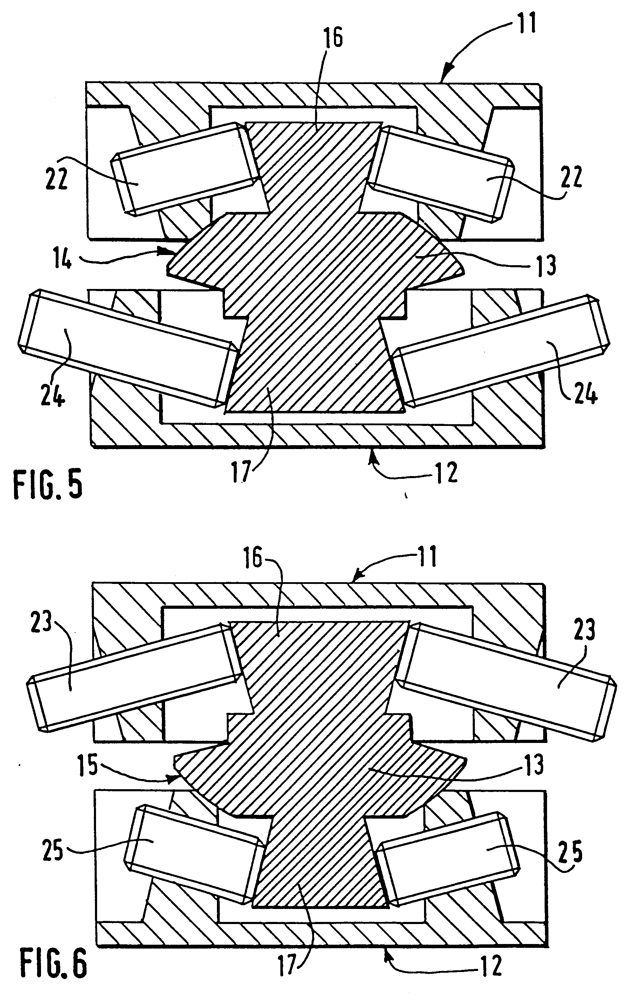

Les figures 4 à 6 représentent une forme de mise en oeuvre préférée de l'invention.

Comme on le voit, le dispositif comprend deux platines, respectivement 11 et 12, aptes à être rendues solidaires, respectivement, de deux parties d'une prothèse, par des moyens usuels, non représentés, et un élément intermédiaire 13, susceptible d'être rendu solidaire, en une pluralité de positions, de chacun des éléments 11 et 12.

L'élément 13 est constitué d'une noix, comprenant deux portions cylindriques 14 et 15, à axes perpendiculaires, à partir de chacune desquelles font saillie, respectivement, deux portions 16 et 17, en forme de tronc de pyramide à base rectangulaire, attenantes par leur petite base à la portion cylindrique associée, avec leur grande base écartée de cette portion cylindrique. Les grands côtés des grandes bases des portions 16 et 17 en forme de tronc de pyramide sont parallèles aux axes des portions cylindriques 14 et 15.

Dans les éléments 11 et 12 sont creusées des parties femelles destinées à recevoir respectivement, d'une part, les portions mâles 14 et 16 de l'élément 13, d'autre part, les portions mâles 15 et 17 de cet élément.

La partie femelle creusée dans l'élément 11 comprend une portion cylindrique 18, de forme complémentaire de celle de la partie 14, et une rainure 19, à section sensiblement parallèlépipédique, dirigée parallèlement à l'axe de la partie cylindrique 14. La dimension transversale de cette rainure 19, c'est-à-dire la dimension perpendiculaire à la direction de l'axe de la partie cylindrique 14, est supérieure à la dimension correspondante de la partie 16 qu'elle est destinée à recevoir. De même, la dimension longitudinale de la rainure 19, c'est-à-dire la dimension parallèle à l'axe de la portion cylindrique 14, est supérieure à la dimension correspondante de la partie 16.

Il est ainsi possible, d'une part, de faire pivoter l'un par rapport à l'autre les éléments 11 et 13, autour des axes des portions cylindriques 14 et 18, pour régler leur position relative angulaire, d'autre part, de faire coulisser l'un par rapport à l'autre les éléments 11 et 13, par déplacement longitudinal de la portion 16 dans la rainure 19, pour régler en translation leurs positions respectives.

De façon analogue, la partie femelle creusée dans l'élément 12 comporte une portion cylindrique 20, de forme complémentaire de celle de la partie 15, et une rainure 21, à section sensiblement paraléllèpipédique, dirigée parallèlement à l'axe de la partie cylindrique 15. La rainure 21 a une longueur et une largeur supérieures aux dimensions correspondantes de la portion 17, ce qui permet de faire pivoter l'une par rapport à l'autre les portions cylindriques 15 et 20 et d'ajuster par translation la position des parties 17 et 21.

Un double réglage en position est donc possible, de façon particulièrement simple, au niveau de chacune des platines 11 et 12, ce qui permet d'ajuster les positions relatives, angulaires et en translation, des parties de prothèse solidaires des platines 11 et 12.

Une fois ce réglage effectué, il suffira de verrouiller dans leur position relative les éléments 11 et 13, d'une part, 12 et 13, d'autre part, pour conserver la position adoptée pour les élément de prothèse. Dans ce but, des évidements filetés seront ménagés transversalement dans les parties 11 et 12, et des vis, 22 et 23, d'une part, 24 et 25, d'autre part, vissées dans ces évidements, viendront respectivement en butée contre les flancs latéraux des parties 16 et 17 en forme de tronc de pyramide.

On notera la grande simplicité du dispositif qui vient d'être décrit.

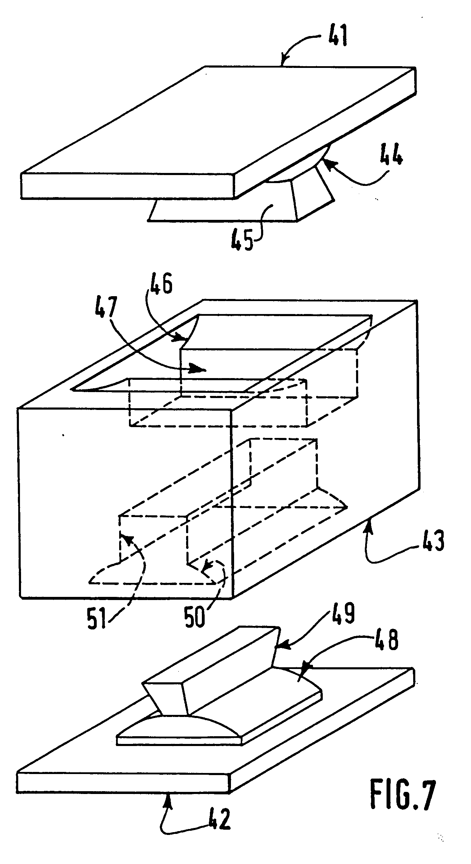

En variante, comme représenté sur la figure 7, les parties mâles peuvent être portées par les éléments 41 et 42 destinés à être rendus solidaires des parties de la prothèse et les parties femelles peuvent être ménagées dans l'élément intermédiaire 43.

La partie mâle de l'élément 41 comporte une portion cylindrique 44 à partir de laquelle fait saillie une portion 45 en forme de tronc de pyramide dont la grande base est éloignée de la portion 44. Cette portion 44 vient coopérer avec une portion femelle 46, de profil complémentaire de l'élément 43, tandis que la portion 45 est logée dans une rainure 47 de cet élément 43, dont les flancs sont sensiblement parallèles à l'axe de la partie cylindrique 44. Les dimensions transversales et longitudinales de la rainure 47 sont supérieures aux dimensions correspondantes de la portion 45, de façon à permettre un pivotement de la portion 44 par rapport à la portion 46 et un coulissement de la portion 45 par rapport à la rainure 47.

De façon analogue, l'élément 42 comporte une portion cylindrique mâle 48, à partir de laquelle fait saillie une portion 49 en forme de tronc de pyramide. L'axe de la portion 48 est perpendiculaire à celui de la portion 44. La portion 48 vient se loger dans une portion femelle 50 de profil complémentaire de l'élément 43, tandis que la portion 49 vient se loger dans une rainure 51, dont les flancs sont parallèles à l'axe de la partie 48 et dont les dimensions longitudinales et transversales sont supérieures à celles de la partie 49, de manière à offrir à celle-ci une double liberté de mouvement, autorisant une rotation de la portion 48 par rapport à la portion 50 et une translation de la portion 49 par rapport à la portion 51.

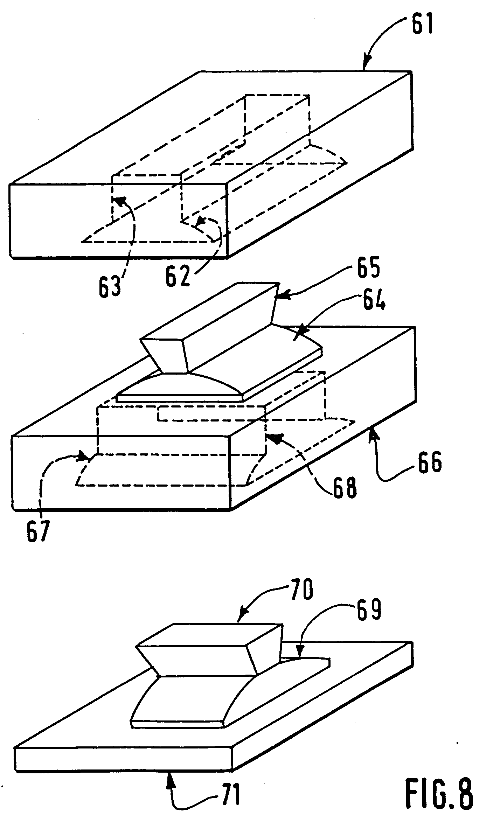

Enfin, comme représenté sur la figure 8, l'un des éléments destinés à être rendus solidaires des parties de la prothèse, l'élément 61 par exemple, peut comporter une partie cylindrique femelle 62 et une rainure 63 aux flancs parallèles à l'axe de la portion 62, destinées à recevoir respectivement une partie cylindrique mâle 64 et une partie en forme de tronc de pyramide 65 de l'élément intermédiaire 66, tandis que, inversement, une partie cylindrique femelle 67 d'axe perpendiculaire à celui de la partie 64 et une rainure 68 de l'élément intermédiaire 66, aux flancs sensiblement parallèles à l'axe de la partie cylindrique 67, recevront respectivement une partie cylindrique mâle 69, de profil complémentaire, et une partie 70 en forme de tronc de pyramide, faisant saillie par rapport à un second élément 71, destiné à être rendu solidaire de l'une des parties de la prothèse. Comme précédemment, les rainures 63 et 68 auront des dimensions transversales et longitudinales supérieures à celles des parties correspondantes 65 et 70.

Dans ces trois formes de réalisation, le réglage en position des trois constituants du dispositif est particulièrement facile à réaliser, mais il est encore possible d'apporter un degré de réglage supplémentaire de la position de ces constituants, en rotation autour d'un axe perpendiculaire à l'axe de pivotement des surfaces cylindriques.

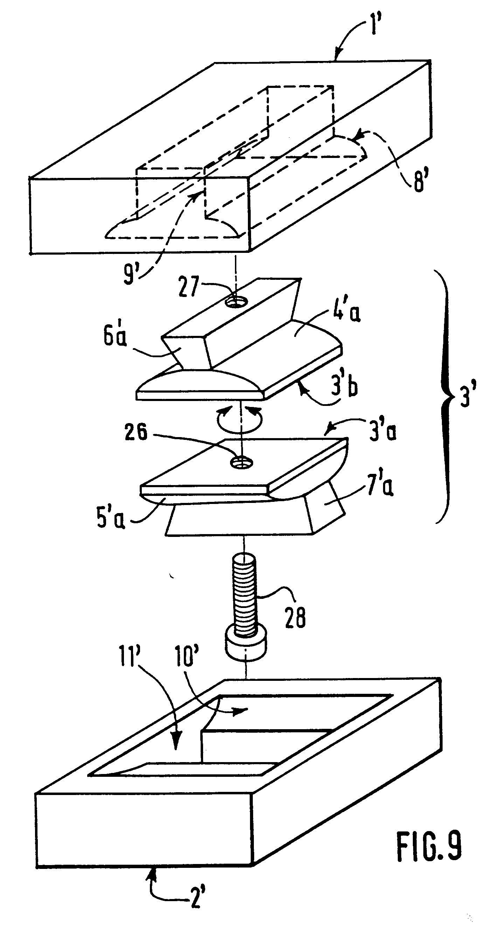

C'est ce qui est illustré par la figure 9, qui est à rapprocher de la figure 4 décrite ci-dessus et sur laquelle les organes déjà décrits en référence à cette figure 4 sont désignés par les mêmes chiffres de référence affectés de l'indice '.

Dans cette forme de réalisation, la pièce intermédiaire 3' est constituée de deux parties 3'a et 3'b en contact par une surface plane parallèle à l'axe des surfaces cylindriques, assemblées entre elles à l'aide d'une vis 28 perpendiculaire à cet axe et vissée dans des évidements filetés 26 et 27, respectivement, de la partie 3'a et de la partie 3'b.

Il est ainsi possible de régler à volonté la position angulaire des parties 3'a et 3'b l'une par rapport à l'autre et de les verrouiller ensuite en position à l'aide de la vis 28.

De façon analogue, on pourrait dédoubler la pièce intermédiaire 43 de la figure 7 en deux parties, en contact mutuel par une surface plane parallèle à l'axe de pivotement des parties cylindriques et assemblées à l'aide d'une vis perpendiculaire à cet axe. Ou encore, on pourrait dédoubler au moins l'une des pièces 32 ou 34 de cette même figure 7 en deux parties en contact mutuel par une surface plane parallèle à l'axe de pivotement des surfaces cylindriques et réunies par une vis perpendiculaire à cet axe.

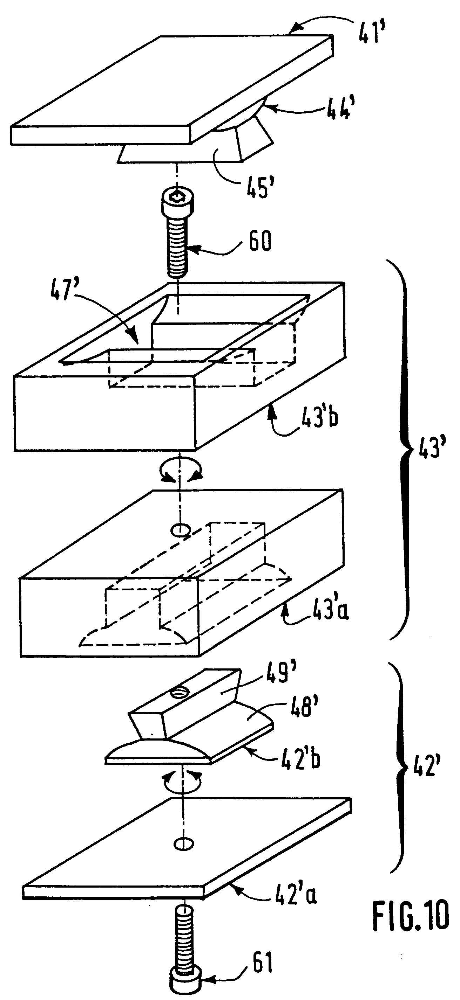

C'est ainsi qu'à titre d'exemple, la figure 10, où les organes déjà décrits en référence à la figure 7 sont désignés par les mêmes chiffres de référence affectés de l'indice ', représente un dispositif dans lequel la partie 43' est constituée de deux parties 43'a, 43'b, comprenant chacune un évidement femelle, en contact mutuel par une surface plane parallèle à l'axe des surfaces cylindriques et assemblées en position réglable en rotation par une vis 60 perpendiculaire à cet axe, tandis que la partie 42' est divisée en deux parties, une platine 42'a et une noix mâle 42'b, en contact mutuel par une surface plane parallèle à l'axe des surfaces cylindriques et assemblées en position réglable en rotation par une vis 61.

Les possibilités additionelles de réglage en position par pivotement d'une pièce l'une par rapport à l'autre ne sont pas limitées à des pièces à surface cylindrique de profil correspondant.

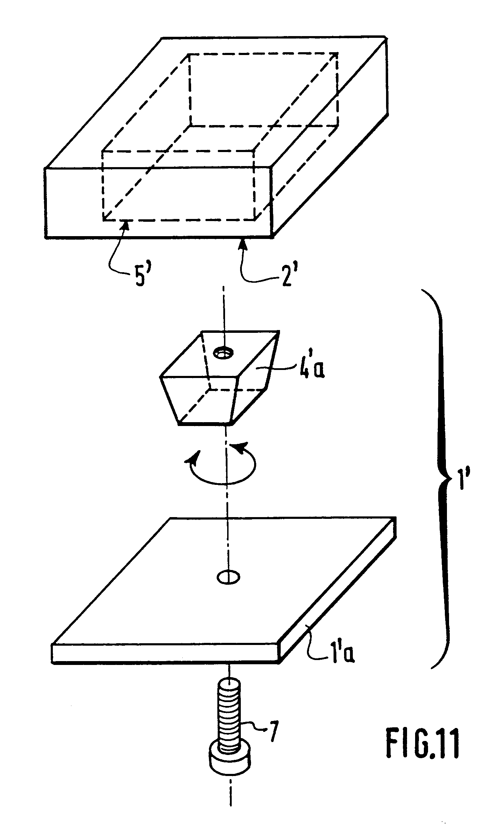

C'est ainsi, par exemple, que dans la réalisation de la figure 11, où les organes déjà décrits en référence à la figure 1 sont désignés par les mêmes chiffres affectés de l'indice', la pièce mâle 1' est constituée de deux parties, une platine 1'a, sur laquelle prend appui la partie femelle 2', et une partie mâle 4'a, les parties 1'a et 4'a étant en contact mutuel par une surface plane et étant assemblées par une vis 7 perpendiculaire à cette surface. L'évidement 5' pourrait naturellement avoir un profil autre que le profil parallélépipédique représenté sur cette figure.

## Revendications

1. Système d'assemblage, en une pluralité de positions réglables, de deux constituants d'une prothèse, dans lequel une partie en saillie (4) solidaire de l'un (1) de ces constituants est engagée dans un évidement formant cuvette (5) de l'autre constituant de la prothèse ou d'un élément intermédiaire (2) de liaison, ou inversement, des vis engagées dans des évidements transversaux filetés de la cuvette (5) étant aptes à venir en butée contre les flancs latéraux de la partie en saillie (4) pour verrouiller celle-ci en position par rapport à la cuvette (5), ce système étant caractérisé en ce que le bord extérieur de l'évidement formant cuvette (5) prend appui de manière à pouvoir glisser de façon rectiligne dans au moins une direction sur une surface du constituant (1) à partir de laquelle la partie (4) fait saillie, tandis que cette partie en saillie (4) est en toute position écartée du fond de l'évidement formant cuvette (5).

2. Système selon la revendication 1, caractérisé en ce que le bord de la cuvette (5) prend appui par au moins une première surface plane contre au moins une autre surface plane de l'élément (1) solidaire de la partie en saillie (4), la première et la seconde surfaces étant disposées de manière à pouvoir glisser relativement l'une par rapport à l'autre.

3. Système selon l'une des revendications 1 et 2, caractérisé en ce que la partie (1'a) solidaire de l'élément en saillie (4'a) et cet élément constituent deux organes distincts en contact par une surface plane contre laquelle prend appui l'élément (2') présentant un évidement formant cuvette (5'), la partie (1'a) et l'élément (4'a) étant assemblés par une vis (7) perpendiculaire à la dite surface plane, en vue de pouvoir régler leur position relative par pivotement par rapport à l'axe de cette vis.

4. Système selon l'une des revendications 1 à 3, caractérisé en ce que les flancs de la partie (4) en saillie sont inclinés par rapport à la surface plane contre laquelle prend appui de manière à pouvoir glisser le bord de l'évidement (5) formant cuvette.

5. Système selon la revendication 4, caractérisé en ce que la partie (4) en saillie a la forme d'un tronc de pyramide inversé, c'est-à-dire dont la plus grande base est éloignée de la partie plane contre laquelle prend appui le bord de l'évidement (5) formant cuvette.

6. Système selon la revendication 1, caractérisé en ce que le bord de la cuvette prend appui par au moins une première surface cylindrique contre au moins une seconde surface cylindrique de profil complémentaire de l'élément dont est solidaire la partie en saillie, les dites première et seconde surfaces cylindriques étant disposées de façon telle qu'elles puissent glisser en pivotant l'une par rapport à l'autre.

7. Système d'assemblage selon la revendication 6, ce dispositif comprenant un premier élément (11), apte à être rendu solidaire d'un premier constituant d'une prothèse, et un second élément (12), apte à être rendu solidaire d'un second constituant de la prothèse, un élément intermédiaire (13), apte à être rendu solidaire, en une position réglable, respectivement, du premier et du second éléments, par coopération d'une partie mâle et/ou d'une partie femelle, respectivement, de cet élément intermédiaire, avec une partie femelle et/ou une partie mâle du premier et du deuxième éléments, des organes d'assemblage tels que des vis étant prévus pour verrouiller l'un par rapport à l'autre, dans une pluralité de positions, d'une part, le premier élément et l'élément intermédiaire, d'autre part, le second élément et l'élément intermédiaire, ce dispositif étant caractérisé en ce que chacune des dites parties mâles comprend une portion en forme de tronc de pyramide (16, 17) à base rectangulaire, faisant saillie à partir d'une portion de forme cylindrique (14, 15), à laquelle il est raccordé par sa petite base, l'axe de la portion cylindrique étant sensiblement parallèle à deux côtés de la base du tronc de pyramide associé, tandis que chaque partie femelle comprend une portion (18, 20) de forme complémentaire de celle de l'élément cylindrique et une portion évidée en forme de rainure (19, 21) apte à recevoir la portion (16, 17) de l'élément mâle associé en forme de tronc de pyramide, la rainure ayant des dimensions supérieures à celles de la portion en forme de tronc de pyramide, parallèlement à l'axe de la portion cylindrique et perpendiculairement à celle-ci, de manière à offrir une double liberté de mouvement, en rotation par rapport à cet axe et en translation parallèlement à cet axe, de la portion mâle par rapport à la portion femelle associée, les parties cylindriques (14, 15) associées à chacun des premier et second éléments ayant en outre des axes non parallèles et, de préférence, perpendiculaires entre eux.

8. Système selon la revendication 7, caractérisé en ce que l'élément intermédiaire (13) comporte deux parties mâles (14, 16 ; 15, 17), associées chacune à deux parties femelles (18, 19 ; 20, 21) des premier et deuxième éléments (11, 12).

9. Système selon la revendication 1, caractérisé en ce que l'élément intermédiaire (43) comporte deux parties femelles (46, 47 ; 50, 51) associées chacune à deux parties mâles (44, 45 ; 48, 49), des premier et deuxième éléments (41, 42).

10. Système selon la revendication 1, caractérisé en ce que l'élément intermédiaire (66) comprend une partie mâle (64, 65) et une partie femelle (67, 68) associées respectivement à une partie femelle (62, 63) et à une partie mâle (69, 70) des premier et deuxième éléments (61, 71).

11. Système selon l'une des revendications 7 à 10, caractérisé en ce que la pièce intermédiaire (3' ; 43') comprend deux parties (3'a, 3'b ; 43'a, 43'b) en contact mutuel par une surface plane parallèle à l'axe de pivotement des surfaces cylindriques, ces deux parties étant assemblées par une vis (28, 61) perpendiculaire à cet axe.

12. Système selon l'une des revendications 7 à 10, caractérisé en ce que au moins l'une des parties mâles (42') comprend une platine (42'a) et une noix mâle (42'b), en contact mutuel par une surface plane parallèle à l'axe de pivotement des surfaces cylindriques associées et assemblées par une vis (61) perpendiculaire à la dite surface plane.

## Claims

1. A system for assembling, in a plurality of adjustable positions, two components of a prosthesis, in which a projecting part (4) firmly with one (1) of these components is engaged in a recess forming a trough (5) in the other component of the prosthesis or an intermediate connecting element (2), or vice versa, screws engaged in threaded transverse recesses in the trough (5) being capable of coming into a position of abutment against the lateral faces of the projecting part (4) to lock the latter in position with respect to the trough (5), this system being characterised in that the outer edge of the recess forming the trough (5) rests, in such a way as to be able to slide rectilinearly in at least one direction, on a surface of the component (1) from which the part (4) projects, while in all positions this projecting part (4) is spaced apart from the base of the recess forming the trough (5).

2. A system according to claim 1, characterised in that the edge of the trough (5) rests with at least a first flat surface against at least one other flat surface of the element (1) firmly with the projecting part (4), the first and second surfaces being arranged in such a way as to be able to slide relative to one another.

3. A system according to either one of claims 1 and 2, characterised in that the part (1'a) firmly connected with the projecting element (4'a) and said element constitute two distinct elements in contact via a flat surface against which there rests the element (2') comprising a recess forming a trough (5'), the part (1'a) and the element (4'a) being assembled by means of a screw (7) perpendicular to said flat surface, with a view to being able to adjust their relative position by pivoting with respect to the axis of this screw.

4. A system according to any one of claims 1 to 3, characterised in that the sides of the projecting part (4) are inclined with respect to the flat surface against which the edge of the recess (5) forming the trough rests in such a way as to be able to slide.

5. A system according to claim 4, characterised in that the projecting part (4) is in the form of an inverted truncated pyramid, that is to say one whose larger base is remote from the flat part against which rests the edge of the recess (5) forming a trough.

6. A system according to claim 1, characterised in that the edge of the trough rests with at least a first cylindrical surface against at least a second cylindrical surface of complementary profile on the element with which the projecting part is firmly connected, said first and second cylindrical surfaces being arranged in such a way that they may slide pivotingly with respect to each other.

7. An assembly system according to claim 6, this device comprising a first element (11), capable of being firmly connected with a first component of a prosthesis, and a second element (12), capable of being firmly connected with a second component of the prosthesis, an intermediate element (13), capable of being firmly connected, in an adjustable position, respectively with the first and second elements through the cooperation of a male part and/or a female part respectively of this intermediate element with a female part and/or a male part of the first and second elements, assembly members such as screws being provided to lock with respect to each other, in a plurality of positions, on the one hand the first element and the intermediate element and on the other hand the second element and the intermediate element, this device being characterised in that each of said male parts comprises a portion in the form of a rectangular-based truncated pyramid (16, 17) projecting from a cylindrically shaped portion (14, 15) to which it is connected by its small base, the axis of the cylindrical portion being substantially parallel with two sides of the base of the associated truncated pyramid, while each female part comprises a portion (18, 20) complementary in shape to the cylindrical element and a recessed portion in the form of a groove (19, 21) capable of receiving the portion (16, 17) of the associated male element in the form of a truncated pyramid, the groove having dimensions greater than those of the portion in the form of a truncated pyramid in parallel with the axis of the cylindrical portion and perpendicularly thereto, in such a way as to provide double freedom of movement, in the form of rotation with respect to this axis and translation in parallel with this axis, of the male portion with respect to the associated female portion, the cylindrical parts (14, 15) associated with each of the first and second elements further having axes which are not parallel and are preferably perpendicular to each other.

8. A system according to claim 7, characterised in that the intermediate (13) comprises two male parts (14, 16; 15, 17), each associated with two female parts (18, 19; 20, 21) of the first and second elements (11, 12).

9. A system according to claim 1, characterised in that the intermediate element (43) comprises two female parts (46, 47; 50, 51) associated respectively with two male parts (44, 45; 48, 49) of the first and second elements (41, 42).

10. A system according to claim 1, characterised in that the intermediate element (66) comprises a male part (64, 65) and a female part (67, 68) respectively associated with a female part (62, 63) and a male part (69, 70) of the first and second elements (61, 71).

11. A system according to any one of claims 7 to 10, characterised in that the intermediate member (3'; 43') comprises two parts (3'a, 3'b; 43'a, 43'b) in mutual contact via a flat surface parallel with the pivot axis of the cylindrical surface, these two parts being assembled by a screw (28, 61) perpendicular to this axis.

12. A system according to any one of claims 7 to 10, characterised in that at least one of the male parts (42') comprises a plate (42'a) and a male plug piece (42'b) in mutual contact via a flat surface parallel with the pivot axis of the associated cylindrical surfaces, said plate (42'a) and male plug piece (42'b) being assembled via a screw (61) perpendicular to said flat surface.

## Patentansprüche

1. Verbindungssystem für zwei Teile einer Prothese, das in einer Vielzahl von Positionen einstellbar ist und bei dem ein fest mit dem einen (1) dieser Teile verbundener abstehender Abschnitt (4) in eine eine Schale (5) bildende Vertiefung des anderen Teiles der Prothese oder eines verbindenden Zwischenelementes (2) eingreift oder umgekehrt, wobei in transversale Vertiefungen mit Innengewinde der Schale (5) Schrauben eingesetzt sind, die dazu geeignet sind, die Seitenflächen des vorstehenden Abschnitts (4) zu beaufschlagen, um diesen in seiner Stellung in Bezug auf die Schale (5) zu verriegeln, dadurch gekennzeichnet, daß der äußere Rand der die Schale (5) bildenden Vertiefung auf einer Fläche des Teiles (1), von dem der Abschnitt (4) absteht, derart zur Auflage kommt, daß er in wenigstens einer Richtung geradlinig auf der Fläche verschiebbar ist, während der abstehende Abschnitt (4) in jeder Position von dem Boden der die Schale (5) bildenden Vertiefung beabstandet ist.

2. Verbindungssystem nach Anspruch 1, dadurch gekennzeichnet, daß der Rand der Schale (5) mit wenigstens einer ersten ebenen Fläche auf wenigstens einer anderen ebenen Fläche des fest mit dem abstehenden Abschnitt (4) verbundenen Elements zur Auflage kommt, wobei die erste und die zweite Fläche gegeneinander gleitfähig angeordnet sind.

3. Verbindungssystem nach einem der Ansprüche 1 und 2, dadurch gekennzeichnet, daß der mit dem abstehenden Element (4'a) fest verbundene Abschnitt (1'a) und dieses Element zwei verschiedene Teile bilden, die über eine ebene Fläche in Kontakt stehen, auf welcher das Element (2') zur Auflage kommt, das eine eine Schale (5') bildende Vertiefung aufweist, wobei der Abschnitt (1'a) und das Element (4'a) mittels einer senkrecht zu der ebenen Fläche verlaufenden Schraube (7) derart verbunden sind, daß ihre relative Position durch Drehung bezüglich der Achse dieser Schraube einstellbar ist.

4. Verbindungssystem nach einem der Ansprüche 1 bis 3, dadurch gekennzeichnet, daß die Flanken des abstehenden Abschnitts (4) in Bezug auf die ebene Fläche, gegen die der Rand der eine Schale bildenden Vertiefung (5) gleitfähig zur Auflage kommt, geneigt sind.

5. Verbindungssystem nach Anspruch 4, dadurch gekennzeichnet, daß der abstehende Abschnitt (4) die Form eines umgekehrten Pyramidenstumpfs aufweist, d.h. dessen größere Grundfläche liegt von dem ebenen Abschnitt entfernt, mit welchem der Rand der die Schale bildenden Vertiefung (5) zur Auflage kommt.

6. Verbindungssystem nach Anspruch 1, dadurch gekennzeichnet, daß der Rand der Schale mit wenigstens einer ersten zylindrischen Oberfläche auf wenigstens einer zweiten zylindrischen Oberfläche mit komplementärem Profil des fest mit dem abstehenden Abschnitt verbundenen Elements zur Auflage kommt, wobei die erste und die zweite zylindrische Oberfläche gegeneinander verdrehbar gleitend angeordnet sind.

7. Verbindungssystem nach Anspruch 6, umfassend ein erstes Element (11), das zum festen Verbinden mit einem ersten Teil einer Prothese geeignet ist, und ein zweites Element (12), das zum festen Verbinden mit einem zweiten Teil der Prothese geeignet ist, ein Zwischenelement (13), das mit jeweils dem ersten und dem zweiten Element in einer einstellbaren Position durch Zusammenwirken eines männlichen bzw. weiblichen Abschnitts des Zwischenelements mit einem weiblichen bzw. männlichen Abschnitts des ersten und des zweiten Elements fest verbindbar ist, Verbindungsorgane wie Schrauben, die zum gegenseitigen Verriegeln des ersten Elements und des Zwischenelements einerseits und des zweiten Elements und des Zwischenelements andererseits in einer Vielzahl von Positionen vorgesehen sind, dadurch gekennzeichnet, daß jeder der männlichen Abschnitte einen pyramidenstumpfförmigen Bereich (16, 17) mit rechteckiger Grundfläche aufweist, der von einem zylinderförmigen Bereich (14, 15) absteht, mit welchem er über seine kleine Grundfläche verbunden ist, wobei die Achse des zylindrischen Abschnitts im wesentlichen parallel zu zwei Seiten der Grundfläche des zugeordneten Pyramidenstumpfes ist, während jeder weibliche Abschnitt einen Bereich (18, 20) mit einer zu dem zylindrischen Element komplementären Form und einen in Form eines Schlitzes (19, 21) vertieften Bereich aufweist, der dazu geeignet ist, den pyramidenstumpfförmigen Bereich (16, 17) des zugehörigen männlichen Elements aufzunehmen, wobei der Schlitz Abmessungen hat, die parallel zu der Achse des zylindrischen Bereichs und senkrecht zu dieser größer sind als diejenigen des pyramidenstumpfförmigen Bereichs, wodurch eine doppelte Bewegungsfreiheit des männlichen Bereichs in Bezug auf den zugehörigen weiblichen Bereichs gegeben ist, nämlich eine Rotation bezüglich dieser Achse und eine Translation parallel zu dieser Achse, wobei die den ersten und zweiten Elementen zugeordneten zylindrischen Bereiche (14, 15) darüber hinaus Achsen aufweisen, die untereinander nicht parallel und, vorzugsweise, senkrecht sind.

8. Verbindungssystem nach Anspruch 7, dadurch gekennzeichnet daß das Zwischenelement (13) zwei männliche Abschnitte (14, 16; 15, 17) umfaßt, die jeweils zwei weiblichen Abschnitten (18, 19; 20; 21) des ersten und des zweiten Elementes (11, 12) zugeordnet sind.

9. Verbindungssystem nach Anspruch 1, dadurch gekennzeichnet daß das Zwischenelement (43) zwei weibliche Abschnitte (46, 47; 50, 51) umfaßt, die jeweils zwei männlichen Abschnitten (44, 45; 48, 49) des ersten und zweiten Elements (41, 42) zugeordnet sind.

10. Verbindungssystem nach Anspruch 1, dadurch gekennzeichnet daß das Zwischenelement (66) einen männlichen Abschnitt (64, 65) und einen weiblichen Abschnitt (67, 68) umfaßt, die jeweils einem weiblichen Abschnitt (62, 63) und einem männlichen Abschnitt (69, 70) des ersten und zweiten Elements (61, 71) zugeordnet sind.

11. Verbindungssystem nach einem der Ansprüche 7 bis 10, dadurch gekennzeichnet, daß das Zwischenstück (3'; 43') zwei Abschnitte (3'a, 3'b; 43'a, 43'b) umfaßt, die über eine zu der Drehachse der zylindrischen Flächen parallele ebene Fläche in gegenseitigem Kontakt stehen und die mittels einer Schraube (28, 61) senkrecht zu dieser Achse miteinander verbunden sind.

12. Verbindungssystem nach einem der Ansprüche 7 bis 10, dadurch gekennzeichnet, daß wenigstens einer der männlichen Abschnitte (42') eine Platte (42'a) und eine männliche Nuß (42'b) umfaßt, die über eine parallel zu der Drehachse der zugehörigen zylindrischen Flächen ebene Fläche in gegenseitigem Kontakt stehen und mittels einer senkrecht zu der ebenen Fläche verlaufenden Schraube (61) miteinander verbunden sind.
